# EUROPEAN PATENT SPECIFICATION

(11) **EP 0 319 170 B1**
(45) Date of publication and mention of the grant of the patent: **17.03.1993**
(21) Application number: 88310862.3
(22) Date of filing: 17.11.1988
(51) Int. Cl.: C07D 239/46, C07D 409/12, A61K 31/505

(54) **Acyl derivatives of hydroxy pyrimidines**
Acyl-Derivate von Hydroxy-Pyrimidinen
Dérivés acylés d'hydroxy pyrimidines

(30) Priority: 02.12.1987 WO PCT/US87/03171
(43) Date of publication of application: 07.06.1989
(73) Proprietor: PFIZER INC., New York, N.Y. 10017 (US)
(72) Inventor: Walker, Frederick Judson, Groton Connecticut (US); Lamattina, John Lawrence, Ledyard Connecticut (US)
(74) Representative: Bradbrook, Geoffrey William

(56) References cited:
- EP-A- 0 210 044

## Description

This invention relates to acyl derivatives of hydroxy substituted pyrimidines, more particularly, 2-amino and 2-substituted amino-4-substituted-5-hydroxy pyrimidines which are optionally 6-substituted and pharmaceutical compositions containing such compounds as active ingredients Hydroxy compounds from which the compounds of the present invention may be prepared and that are inhibitors of leukotriene synthesis are disclosed in European Patent Application publication No. 0 210 044.

Current treatment of asthma focuses on the relief of acute bronchospasm through the use of bronchodilators. It is thought that acute bronchospasm is only an overt manifestation of chronic inflammation. Leukotrienes may play a role both in the bronchospasm and the chronic inflammation. They are known to be potent vasodilators and chemotactic agents. They are also produced in allergic reactions and bring about slow contraction of lung tissue in vitro. An inhibitor of leukotriene synthesis should therefore be of use in the treatment of asthma and other pulmonary diseases.

Chronic gastric and duodenal ulcers, together known as peptic ulcers, are the subject of a variety of treatments, including special diets, drug therapy and surgery, depending upon the severity of the condition. Particularly valuable therapeutic agents useful for the treatment of gastric hyperacidity and peptic ulcers are the histamine-H₂ receptor antagonists, which block the action of the physiologically-active compound histamine at the H₂-receptor sites in the animal body and thereby inhibit the secretion of gastric acid.

In accordance with one aspect of the invention, there are provided substituted pyrimidines having the formula: 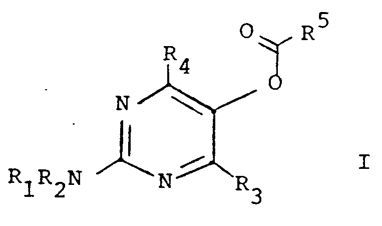 wherein R₁ is hydrogen or (C₁-Cₗ₅)alkyl; R₂ is hydrogen, (C₁-Cₗ₅)alkyl, cyclopentyl, cyclohexyl, (C₃-Cₗ₅)-alkenyl, phenyl, substituted phenyl, (C₇-C₂₀)phenylalkyl, or substituted (C₇-C₂₀)phenylalkyl; or R₁ and R₂ together with the nitrogen atom to which they are attached form a pyrrolidinyl or piperidyl group which may be substituted by one (C₁-C₆)alkyl, phenyl or (C₇-C₂₀)phenylalkyl; R₃ is (C₁-C₆)alkyl, phenyl, substituted phenyl, (C₇-C₂₀)phenylalkyl, substituted (C₇-C₂₀)phenylalkyl, furyl, thienyl, furyl substituted by one (C₁-C₃)-alkyl, or thienyl be substituted by one (C₁-C₃)alkyl; R₄ is hydrogen, (C₁-C₆)alkyl, phenyl, substituted phenyl, (C₇-C₂₀)phenylalkyl, or substituted (C₇-C₂₀)phenylalkyl; and R₅ is hydrogen, (C₁-C₁₀)alkyl, (C₁-C₁₀)alkoxy, phenyl, substituted phenyl, (C₇-C₂₀)phenylalkyl, substituted (C₇-C₂₀)phenylalkyl, (C₂-C₃)alkylcarboxy, -NR₆R₇, wherein R₆ and R₇ are independently selected from the group consisting of (C₁ -C₁₀ )alkyl, phenyl, substituted phenyl, (C₇-C₂₀)phenylalkyl and substituted (C₇-C₂₀)phenylalkyl; wherein the phenyl moieties on said substituted phenyl and said substituted phenylalkyl are substituted by one or two moieties selected from the group consisting of fluoro, chloro, (C₁-C₃)alkyl, (C₁-C₃)alkoxy and CF₃; with the proviso that when R₁ and R₂ are both hydrogen, R⁵ cannot be methyl and with the further proviso that when R³ and R⁵ are methyl and R⁴ is isopropyl, R¹ and R² are not both methyl and R² is not p-chlorophenylpropyl when R¹ is H, pharmaceutically acceptable acid addition salts thereof, and, when R₅ is (C₂-C₃)alkylcarboxy, pharmaceutically acceptable base addition salts thereof.

A preferred embodiment of the present invention relates to compounds of formula I wherein R₁ and R₅ are as defined above, R₂ is hydrogen, (C₁-Cₗ₅)alkyl, phenyl, substituted phenyl, (C₇-C₂₀)phenylalkyl, or substituted (C₇-C₂₀)phenylalkyl,wherein the phenyl moieties on said substituted phenyl and said substituted phenylalkyl may be substituted by one or two moieties selected from the group consisting of chloro and (C₁-C₃)alkyl; R₃ is (C₁-C₆)alkyl, phenyl which may be substituted by one or two moieties selected from the group consisting of fluoro, chloro, methyl, ethyl, methoxy, ethoxy, and CF₃; and R₄ is hydrogen, (C₁-C₆)-alkyl, phenyl or phenyl substituted by one or two moieties selected from the group consisting of methyl and ethyl; the pharmaceutically acceptable acid addition salts thereof, and, when R₅ is (C₂-C₃)alkylcarboxy, the pharmaceutically acceptable base addition salts thereof. More preferably, R₁ is hydrogen and R₂ is other than hydrogen.

Another preferred embodiment of the present invention relates to compounds of the formula I wherein R¹ is hydrogen; R² is (C₇-Cₗ₂)phenylalkyl which may be substituted in the phenyl moiety by one or two moieties selected from the group consisting of fluoro, chloro, (C₁-C₃)alkyl, (C₁-C₃)alkoxy, and CF₃; R₃ and R₄ are each methyl; and R₅ is as defined above; pharmaceutically acceptable acid addition salts thereof, and, when R₅ is (C₂-C₃)alkylcarboxy, pharmaceutically acceptable base addition salts thereof.

A particularly preferred embodiment of the present invention relates to compounds of the formula I wherein R₁ is hydrogen; R₂ is (C₃-C₃) alkyl, (C₉-C₁₂)phenylalkyl, (C₉-Cₗ₂)p-chlorophenylalkyl or (C_{1O}-C₁₃) p-methylphenylalkyl; R₃ and R₄ are each methyl; and R₅ is (Cᵢ-C₆)alkyl or (Cᵢ-C₆)alkoxy; and the pharmaceutically acceptable acid addition salts thereof.

Specifically preferred compounds of the present invention are as follows:
5-Acetoxy-4,6-dimethyl-2-phenylhexylaminopyrimidine; and
4,6-Dimethyl-2-phenylhexylamino-5-(ethyloxyformyl)-oxypyrimidine.

The present invention also relates to the title compounds of Examples 2-11 and 13-19 set forth below.

Specific compositions of the invention contain the specific compounds mentioned above and preferred compositions contain the preferred compounds mentioned above.

The invention further includes these compounds for use in medicine including treating a mammal affected by pulmonary, asthmatic, allergic or inflammatory diseases with a compound as defined above or a pharmaceutically acceptable acid addition salt thereof.

The term "alkyl" in the definitions of groups Ri, R₂, R₃, R₄, R₅ and R₆ denotes saturated monovalent straight or branched aliphatic hydrocarbon radicals such as methyl, ethyl, propyl, butyl, t-butyl, hexyl, octyl, 2-ethylhexyl etc.

The term "phenylalkyl" in the definitions of groups R₂, R₃, R₄ and R₅ denotes a phenyl group attached to saturated divalent straight or branched aliphatic hydrocarbon radicals. Examples of such phenylalkyls are methylphenyl, ethylphenyl, propylphenyl, butylphenyl, pentylphenyl, hexylphenyl, octylphenyl, 1,1-dimethyl-7-phenylheptyl etc.

The compounds of the present invention may be prepared by acylating a compound of the formula 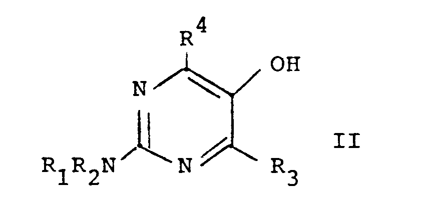 wherein Ri, R₂, R₃ and R₄ are as defined for formula I with an acylating agent. Compounds of the formula II are disclosed in European Patent Application Publication No. 0 210 044.

The acylating agent may be an active ester, for example, an anhydride (e.g., acetic anhydride or succinic anhydride) or an acid chloride. Thus, for example, the acylating agent may be a compound of the formula 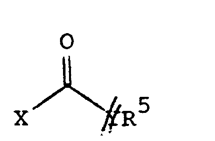 wherein X is chlorine or bromine, and R⁵ is as defined for formula I. Alternatively, the acylating agent may be a compound of the formula 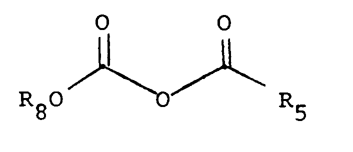 wherein R⁸ is (C1 -C6 )alkyl (e.g., isobutyl) and R₅ is as defined for formula I, except that R₅ cannot be alkoxy or aminoalkyl.

In preparing a compound of the present invention, the acylating agent may be reacted with the hydroxypyrimidine of the formula II in an inert solvent, such as methylene chloride or ether, in the presence of a base, such as triethylamine or pyridine, under a dry inert atmosphere, such as dry nitrogen or dry argon. Alternatively, a base, such as pyridine, may be used as the solvent. The reaction mixture should preferably be maintained at a temperature of about -20 to about 50 ° C, more preferably at about 0 ° C, for about 0.5 to about 24 hours, generally for about 2 hours. After acylation, the product may be treated with an acid, such as phosphoric acid, to form an acid addition salt, or the product may be treated with a base, such as sodium hydroxide, to form a base addition salt.

The acid addition salts of the compounds of formula I are prepared in a conventional manner by treating a solution or suspension of the free base (I) with about one chemical equivalent of a pharmaceutically acceptable acid. Conventional concentration and recrystallization techniques are employed in isolating the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic such as methanesulfonic, benzensulfonic, and related acids. Preferably, the acid is phosphoric acid.

The base addition salts of the compounds of formula I wherein R₅ is (C₂-C₃)alkylcarboxy may be prepared in a conventional manner by reacting such compounds of the formula I with about one chemical equivalent of an inorganic base such as an alkali metal hydroxide or an alkaline earth metal hydroxide.

The compounds of formula I and their pharmaceutically acceptable acid addition salts are inhibitors of leukotriene synthesis and agents for the treatment of various pulmonary, gastrointestinal, allergic, inflammatory, dermatological and cardiovascular conditions. In particular, the compounds have utility, both as the sole active agent and also in combination with other active agents, for the treatment of mammals, including humans, affected with asthma, bronchitis, pulmonary diseases such as pulmonary hypertension and hypoxia, peptic ulcers, psoriasis, arthritis, inflammatory bowel disease or cardiovascular spasm, such as acute myocardial infarctions.

While we do not wish to be bound by theory, we believe that the compounds of the present invention are metabolized in vivo to form the corresponding 5-hydroxy comounds.

For treatment of the various conditions described above, the compounds of formula I may be administered to a subject in need of treatment by a variety of conventional routes of administration, including oral, by injection, topical, and in an aerosol carrier composition for administration by breathing.

In general, a therapeutically-effective dose for the active compounds of formula I will range from 0.01 to 100 mg/kg body weight of the subject to be treated per day, preferably 0.1 to 50 mg/kg per day.

Although the compounds of formula I can be administered alone, they will generally be administered in admixture with a pharmaceutical carrier selected with regard to the intended route of administration and standard pharmaceutical practice. For example, oral administration may be in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water. For parenteral injection, they may be used in the form of a sterile aqueous solution which may contain other solutes, for example enough salt or glucose to make the solution isotonic. For topical use, they may be formulated in solutions, suspensions, gels, creams, or ointments, such formulations preferably including one or more excipients to prevent or retard decomposition, such as ascorbic acid, sodium bisulfite, or dithiothreitol and agents to adjust the pH, such as sodium hydroxide, hydrochloric acid or sodium bicarbonate.

The activity of the compounds of formula I in the treatment of pulmonary (e.g., asthmatic), allergic, dermatological (e.g., psoriasis) and inflammatory diseases may be determined by a standard test measuring an agent's ability to inhibit cyclooxygenase and lypoxygenase enzyme activity of rat basophil leukemia (RBL-1) cells. According to this test as described by Jakschick et al., Prostaglandins, 16,733-747 (1978), a monolayer of RBL-1 cells is grown for 1 or 2 days in spinner culture in Eagle's minimum essential medium, 15% heat-inactivated fetal calf serum and an antibiotic/antimycotic mixture. The cells are washed after centrifugation and incubated in a buffer. A volume of 0.5 ml of cell suspension is preincubated at 30°C for ten minutes with a 1 µl dimethylsulfoxide (DMSO) solution of the agent to be tested. The incubation is initiated by simultaneous addition of 5 µl ('⁴C)- arachidonic acid in ethanol and 2 /1.1 calcium ionophore (A-21387) in DMSO for final concentrations of 5 and 7.6 M, respectively. Five minutes later, the incubation is terminated by the addition of 0.27 ml acetonitrile/acetic acid (100:3). Thin layer chromatography is performed using acetonitrile/water/acetic acid solvent.

The following Examples illustrate the invention. All melting points referred to in the Examples are uncorrected.

### Example 1

### 5-Acetoxy-4,6-dimethyl-2-phenylhexylaminopyridimidine

4,6-Dimethyl-5-hydroxy-2-phenylhexylaminopyrimidine (0.53 g, 1.77 mmol) was dissolved in dry methylene chloride (10 ml) and treated sequentially with triethyl amine (0.25 ml) and acetic anhydride (0.18 g 1.77 mmol) while chilling to 0°C, under a nitrogen atmosphere. After being stirred for 18 hours, the reaction mixture was concentrated on a rotary evaporator. The residue was dissolved in ethyl acetate (50 ml) and extracted three times with water (75 ml) and one time with brine (50 ml). The dried organic extracts were filtered and concentrated on a rotary evaporator and chromatographed on silica gel, eluted with 1:3, ethyl acetate: hexanes to afford 0.45 g of product, mp: 63-65 ° C. Mass spectrum m/e = 341. NMR (CDCl₃) delta: 7.01 (bs, 5H); 5.03-4.78 (m, 1 H); 3.41-3.15 (m, 2H); 2.70-2.41 (M, 2H); 2.2 (s 3H); 2.09 (s, 6H); 1.71-1.43 (m, 10H). IR (CHCl₃): 3445, 1763, 1763, 1583, 1517 cm-¹.

### Example 2

### 5-Benzoyloxy-4,6-dimethyl-2-phenylhexylaminopyrimidine

4,6-Dimethyl-5-hydroxy-2-phenylhexylaminopyrimidine (0.5 g, 1.7 mmol) was dissolved in pyridine (20 ml), cooled to 0 ° C under nitrogen and treated with benzoyl chloride (0.2 ml, 1.7 mmol). After being stirred for 2 hours the reaction mixture was poured into water (100 ml) and extracted with ethyl acetate (3x25 ml). The combined organics were dried and concentrated and the residue chromatographed to afford 0.41 g of product, mp: 79-80°C. Mass spectrum m/e = 403. NMR (CHCl₃) delta: 8.23 (d, 5 Hz, 2H); 7.62-7.10 (m, 8H); 5.04-4.98 (m, 1H); 3.36 (q, 7, 3 Hz, 2H); 2.56 (t, 5 Hz, 2H); 2.18 (s, 6H); 1.68-1.30 (m, 8H). IR (KBr): 1740, 1600, 1580, 1550 cm^{-1.} Anal. Calcd. for C₂₅H₂₉N₃O₂: C, 74.41; H, 7.24; N, 10.41. Found: C, 74.46; H, 7.04; N, 10.17.

### Example 3

### 4,6-Dimethyl-2-phenylhexylamino-5-o-toluyloxypyrimidine

In a manner similar to that of the method of Example 2, the title compound was prepared from 0.75 g (2.5 mmol) of 4,6-dimethyl-2-phenylhexylamino-5-hydroxypyrimidine to afford 0.25 g of product, mp: 52-53 °C. Mass spectrum m/e = 417. NMR (CDCl₃) delta: 8.15 (d, 4 Hz, 1H); 7.46-7.10 (m, 8H); 4.98 (bs, 1H); 3.37 (q, 5, 3 Hz, 2H) 2.66 (s, 3H); 2.60 (t, 4 Hz, 2H), 2.23 (s, 6H); 1.68-1.30 ( m, 8H). IR (CHCl₃): 1740, 1580 cm-^{1.} Anal. Calcd. for C₂₆ H₃₁ N₃ O₂: C, 74.79; H, 7.48; N, 10.06. Found: C, 74.65; H, 7.55; N, 10.10.

### Example 4

### 5-Caproyloxy-4,6-dimethyl-2-phenylhexylaminopyrimidine

In a manner similar to that of the method of Example 2, the title compound was prepared from 0.75 g (2.5 mmol) of 4,6-dimethyl-5-hydroxy-2-phenylhexylaminopyrimidine to afford 0.71 g of product, mp: 44-45 °C. Mass spectrum m/e = 383. NMR (CDCl₃) delta: 7.26-7.10 (m, 5H); 5.03 (bs, 1H); 3.32 (q, 5, 3 Hz, 2 H); 2.62-2.48 ( m, 4H); 2.14 (s, 6H); 1.78-0.88 (m, 15 H). IR (CHCl₃): 1750, 1600, 1580 cm-¹. Anal. Calcd. for C₂₃H₃₃N₃0₂: C, 72.03; H, 8.67; N, 10.96. Found: C, 71.85; H, 8.65; N, 10.69.

### Example 5

### 4,6-Dimethyl-5-5-phenylpentanoyloxy 2-phenylhexylaminopyrimidine

In a manner similar to that of the method of Example 2, the title compound was prepared from 0.75 g (2.5 mmol) of 4,6-dimethyl-5-hydroxy-2-phenylhexylaminopyrimidine to afford 0.8 g of product, mp: 72-73 ° C. Mass spectrum m/e = 459. NMR (CDCl₃) delta: 7.28-7.06 (m, 10H); 4.88 (bs, 1 H); 3.32 (q, 5, 3 Hz, 2H); 2.68-2.50 ( m, 6H); 2.12 (s, 6H); 1.84-1.28 (m, 12H). IR (KBr): 1750, 1600 1580 cm-¹. Anal. Calcd. for C₂₉H₃₇N₃O₂: C, 75.78; H, 8.11; N, 9.14. Found: C, 75.46; H, 7.87; N, 8.99.

### Example 6

### 4,6-Dimethyl-5-[p-N,N-diethylaminomethyl)]-benzoyloxy-2-phenylhexylaminopyrimidine

In a manner similar to that of the method of Example 1, the title compound was prepared from 0.75 g (2.5 mmol) of 4,6-dimethyl-5-hydroxy-2-phenylhexylaminopyrimidine to afford 0.62 g of product, mp: 49-51 ° C. Mass spectrum m/e = 488. NMR (CDCl₃): delta 8.10 (d, 4 Hz, 2H); 7.47 (d, 4 Hz, 2H); 7.30-7.06 (m, 5H); 4.92 (bs, 1 H); 3.62 (s, 2H); 3.35 (m, 2H); 2.64-2.44 (m, 6H); 2.20 (s, 6H); 1.70-1.28 (m, 8H); 1.04 (t, 3 Hz, 6H). IR (CHCl₃): 1740, 1580 cm-¹. Anal. Calcd. for C₃₀H₄₀N₄O₂: C, 73.74; H, 8.25; N, 11.47. Found: C, 73.40; H, 8.25; N, 11.25.

### Example 7

### 4,6-Dimethyl-2-phenylhexylamino-5-succinoyloxypyrimidine

In a manner similar to that of the method of Example 1, the title compound was prepared from 0.75 g (2.5 mmol) of 4,6-dimethyl-5-hydroxy-2-phenylhexylaminopyrimidine and succinic anhydride (0.25 g, 2.5 mmol) to afford 0.45 g of product, mp: 171-172°C. Mass spectrum m/e = 399. NMR (DMSO-d₆) delta: 7.24-6.90 (m, 6H); 3.21-3.09 (m, 2H); 2.80-2.48 (m, 6H); 2.04 (s, 6H); 1.58-1.22 (m, 8H). IR (KBr): 1700, 1595 cm-^{1.} Anal. Calcd. for C₂₂H₂₉O₄N₃: C, 66.15; H, 7.32; N, 10.52. Found: C, 65.75; H, 7.24; N, 10.41.

### Example 8

### 4,6-Dimethyl-5-[(p-morpholinomethyl)]-benzoyloxy-2-phenylhexylaminopyrimidine

In a manner similar to that of the method of Example 1, the title compound was prepared from 0.78 g (2.33 mmol) of 4,6-dimethyl-5-hydroxy-2-phenylhexylaminopyrimidine and p-morpholinomethylbenzoyl chloride (2.8 mmol) to afford 0.43 g of product, mp: 81-84°C. Mass spectrum m/e = 502. NMR (CDCl₃) delta: 8.00 (d, 4 Hz, 2H); 7.35 (d, 4 Hz, 2H); 7.20-7.00 (m, 5H); 4.9 (m, 1 H); 3.70-3.30 (m, 8H); 2.58-2.30 (m, 4H); 2.16 (s, 6H); 1.64-1.28 (m, 8H). IR (CHCl₃): 1739, 1582 cm-^{1.} Anal. Calcd. for C₃₀H₃₈N₄O₃: C, 71.65; H, 7.62; N, 11.15. Found: C, 71.83; H, 7.70; N,10.98.

### Example 9

### 4,6-Dimethyl-2-phenylhexylamino-5-propanoyloxypyrimidine

In a manner similar to that of the method of Example 2, the title compound was prepared from 0.94 g (3.12 mmol) of 4,6-dimethyl-5-hydroxy-2-phenylhexylaminopyrimidine to afford 0.76 g of product, mp: 66-67 C. Mass spectrum m/e = 355. NMR (CDCl₃) delta: 7.21-7.01 (m, 5H); 4.95 (bs, 1 H); 3.34-3.21 (m, 2H); 2.61-2.42 (m, 4 R); 2.12 (s, 6H); 1.64-1.21 (m, 11H). IR (CHCl₃): 1756, 1579 cm-¹. Anal. Calcd. for C₂₁ H₂₉ N₃ O₂: C, 70.75; H, 8.22; N, 11.82. Found: C, 70.99; H, 7.92; N, 11.91.

### Example 10

### 4,6-Dimethyl-2-phenylhexylamino-5-(2-thienoyl)oxypyrimidine

In a manner similar to that of the method of Example 2, the title compound was prepared from 0.88 g (2.95 mmol) of 4,6-dimethyl-5-hydroxy-2-phenylhexylaminopyrimidine to afford 0.83 g of product, mp: 91-93 °C. Mass spectrum m/e = 409. NMR (CDCl₃) delta: 7.91 (d, 2 Hz, 1H); 7.62 (d, 2 Hz, 1H); 7.24-7.10 (m, 6H); 4.97 (bs, 1H); 3.32 (q, 7, 5 Hz, 2H); 2.57 (t, 4 Hz, 2H); 2.19 (s, 6H); 1.64-1.30 (m, 8H). IR (CHCl₃): 1729, 1580 cm-^{1.} Anal. Calcd. for C₂₃H₂₇N₃0₂S: C, 67.45; H, 6.65; N, 10.26. Found: C, 67.62; H, 6.76; N, 10.10.

### Example 11

### 4,6-Dimethyl-2-phenylhexylamino-5-(phenyloxyformyl)oxypyrimidine

In a manner simlar to that of the method of Example 2, the title compound was prepared from 0.76 g (2.6 mmol) of 4,6-dimethyl-5-hydroxy-2-phenylhexylaminopyrimidine and phenyl chloroformate (0.40 g, 2.6 mmol) to afford 0.74 g of product, mp: 76-77° C. Mass spectrum m/e = 419. NMR (CDCl₃) delta: 7.4-6.9 (m, 10H); 4.99 (bs, 1H); 3.3 (q, 7, 4 Hz, 2H); 2.5 (t, 4 Hz, 2H); 2.3 (s, 6H); 1.8-1.1 (m, 8H). IR (CHCl₃): 1780, 1583 cm-^{1.} Anal. Calcd. for C₂₅H₂₉N₃0₃: C, 71.57.; H, 6.97; N, 10.02. Found: C, 71.51; H, 6.95; N, 10.09.

### Example 12

### 4,6-Dimethyl-2-phenylhexylamino-5-(ethyloxyformyl)oxypyrimidine

A. In a manner similar to that of Example 1, the title compound was prepared from 4.71 g (12 mmol) of 4,6-dimethyl-5-hydroxy-2-phenylhexylaminopyrimidine phosphate salt, triethylamine (16.52 ml, 120 mmol) and ethyl chloroformate (1.7 ml, 18 mmol) to afford 3.8 g of product, mp: 44-45 °C. Mass spectrum m/e = 371. NMR (CDCl₃) delta: 7.34-7.16 (m, 5H); 5.00 (bs, 1H); 4.36 (q, 6, 3 Hz, 2H); 3.40 (q, 6, 3 Hz, 2H); 2.65, (t, 3 Hz, 2H); 1.74-1.36) (m, 11H). IR (CHCl₃): 1761, 1583 cm-^{1.} Anal. Calcd. for C₂₁ H₂₉ N₃O₃: C, 67.90; H, 7.87; N, 11.31. Found: C, 67.95; H, 7.85; N, 11.14.

B. The title compound (0.1 g, 0.27 mmol) was dissolved in isopropanol (5 ml), cooled to 0° C and treated with phosphoric acid (0.031 g, 0.27 mmol). After being stirred for 1 hour, the reaction mixture was allowed to come to 25 ° C, the solvent was removed on a rotary evaporator and the product was recrystallized from ethyl acetate to provide the phosphoric acid addition salt of the title comound, mp: 105-107 C. Anal. Calcd. for C₂₁ H₂₉ O₃ H₃ H₃ PO₄: C, 53.73; H, 6.87; N, 8.95. Found: C, 53.66; H, 6.84; N, 8.80.

### Example 13

### 4,6-Dimethyl-2-phenylhexylamino-5-(isobutyloxyformyl)oxypyrimidine

In a manner similar to that of the method of Example 2, the title compound was prepared from 0.7 g (2.3 mmol) of 4,6-dimethyl-5-hydroxy-2-phenylhexylaminopymidine and isobutyl chloroformate (0.31 ml, 2.3 mmol) to afford 0.79 g of product. Mass spectrum m/e = 399. NMR (CDCl₃) delta: 7.28-7.10 (m, 5H); 4.86 (bs, 1 H); 4.05 (d, 4 Hz, 2H); 3.39 (q, 4, 6 Hz, 2H); 2.58 (t, 5 Hz, 2H); 2.22 (s, 6H); 2.10-1.96 (m, 1 H); 1.70-1.30 (m, 8H); 0.99 (d, 4 Hz, 6H). IR (CHCl₃): 1760, 1580 cm-^{1.} Anal. Calcd. for C₂₃H₃₃0₃N₃: C, 69.14; H, 8.32; N, 10.52. Found: C, 69.20; H, 8.12; N, 10.89.

### Example 14

### 5-(N,N-Dimethylaminoformyl)oxy-4,6-dimethyl-2 phenylhexylaminopyrimidine

In a manner similar to that of the method of Example 2, the title compound was prepared from 0.5 g. (1.7 mmol) of 4,6-dimethyl-5-hydroxy-2-phenylhexylaminopyrimidine and N,N-dimethylcarbamoyl chloride (0.15 ml, 1.7 mmol) to afford 0.55 g of product, mp: 81-82°C. Mass spectrum m/e = 370. NMR (CDCl₃) delta: 7.30-7.10 (m, 5H); 4.88 (bs, 1 H); 3.34 (q, 4, 6 Hz, 2 H); 3.11 (s, 3H); 3.00 (s, 3H); 2.58 (t, 5 Hz, 2H); 2.20 (s, 6H); 1.68-1.32 (m, 8H). IR (CHCI₃): 1730, 1590 cm-^{1.} Anal. Calcd. for C₂₁H₃₀O₂N₄: C, 68.08; H, 8.16; N, 15.12. Found: C, 68.08; H, 7.99; N, 14.75.

### Example 15

### 5-(N,N-Dimethylaminoformyl)oxy-4-isopropyl-6-methyl-2-phenylhexylaminopyrimidine

In a manner similar to that of the method of Example 2, the title compound was prepared from 0.45 g (1.4 mmol) of 5-hydroxy-4-isopropyl-6-methyl-2-phenyl hexylaminopyrimidine and N,N-dimethylcarbamoyl chloride (0.13 ml, 1.4 mmol) to afford 0.4 g of product, mp: 41-42°C. Mass spectrum m/e = 398. NMR (CDCL₃) delta: 7.25-7.04 (m, 5H); 4.83 (bs, 1H); 3.33 (q, 5, 3 Hz, 2H); 3.10 (s, 3H); 3.00 (s, 3H); 2.85-2.97 (m, 1H); 2.57 (t, 4 Hz, 2H); 2.16 (s, 3H); 1.68-1.30 (m, 8H); 1.15 (d, 4 Hz, 6H). IR (CHCI₃): 1725, 1580 cm-^{1.} Anal. Calcd. for C₂₃H₃₄O₂N₄: C, 69.32; H, 8.60; N, 14.06. Found: C, 69.43; H, 8.50; N, 13.75.

### Example 16

### 4,6-Dimethyl-5-pivaloyloxy 2-phenylpentylaminopyrimidine

In a manner similar to that of the method of Example 2, the title compound was prepared from 0.5 g (1.8 mmol) of 4,6-dimethyl-5-hydroxy-2-phenylpentylaminopyrimidine and pivaloyl chloride (0.22 ml, 1.8 mmol) to afford 0.33 g of product, mp: 79-80° C. Mass spectrum m/e = 369. NMR (CDCl₃) delta: 7.32-7.14 (m, 5H); 5.48 (bs, 1 H); 3.36 (q, 5, 3 Hz, 2H); 2.61 (t, 4Hz, 2H); 2.15 (s, 6H); 1.67-1.42 (m, 6H); 1.40 (s, 9H). IR (CHCl₃): 1750, 1580 cm-¹ Anal. Calcd. for C₂₂H₃₁0₂N₃: C, 71.51; H, 8.46; N, 11.37 Found: C, 71.06; H, 8.31; N, 10.94.

### Example 17

### 4,6-Dimethyl-2-phenylpentylamino-5-(isobutyloxyformyl)oxypyrimidine

In a manner similar to that of the method of Example 2, the title compound was prepared from 0.5 g (1.8 mmol) of 4,6-dimethyl-5-hydroxy-2-phenylpentylaminopyrimidine and isobutylchoroformate (0.23 ml, 1.8 mmol) to afford 0.45 g of product. Mass spectrum m/e = 385. NMR (CDCl₃) delta: 7.27-7.16 (m, 5H); 5.00 (bs, 1 H); 4.06 (d, 5 Hz, 2H); 3.00-3.60 (m, 2H); 2.61 (t, 4 Hz, 2H); 2.23 (s, 6H); 1.65-1.36 (m, 6H); 0.99 (d, 4 Hz, 6H). IR (CHCl₃): 1760, 1580 cm-¹. Anal. Calcd. for C₂₂ H₃₁ O₃ N₃: C, 68.54; H, 8.10; N, 10.90. Found: C, 68.14; H, 7.85; N, 10.69.

### Example 18

### 5-Benzoyloxy-4,6-dimethyl-2-phenylhexylaminopyrimidine

In a manner similar to that of the method of Example 2, the title compound was prepared from 0.5 g (1.8 mmol) of 4,6-dimethyl-5-hydroxy-2-phenylpentylaminopyrmidine and benzoyl chloride (0.2 ml, 1.8 mmol) to afford 0.43 g of product, mp: 51-53°C. Mass spectrum m/e = 389. NMR (CDCL₃) delta: 8.22 (d, 5 Hz, 2H); 7.66 (t, 2 Hz, 1 H); 7.53 (t, 2 Hz, 2H); 7.30-7.19 (m, 5 H); 5.09 (bs, 1 H); 3.39 (q, 7, 3 Hz, 2H); 2.61 (t, 5 Hz, 2H); 2.22 (s, 6H); 1.70-1.38 (m, 6H). IR (CHCl₃): 1735, 1570 cm-^{1 .} Anal. Calcd. for C24H2702N3: C, 74.01; H, 6.99; N, 10.79. Found: C, 74.52; H, 7.04; N, 10.73.

### Example 19

### 5-(N,N-Dimethylaminoformyl)oxy-4,6-dimethyl-2-phenylpentylaminopyrimidine

In a manner similar to that of the method of Example 2, the title compound was prepared from 1.0 g (3.6 mmol) of 4,6-dimethyl-5-hydroxy-2-phenylpentylaminopyrmidine and N,N-dimethylaminocarbamoyl chloride (0.32 ml, 3.6 mmol) to afford 0.55 g of product, mp: 63-64 °C. Mass spectrum m/e = 356. IR (CHCl₃): 1720: 1720, 1580 cm-^{1.} Anal. Calcd. for C₂₀ H₂₈ O₂ N₄: C, 67.39; H, 7.92; N, 15.72. Found: C, 66.95; H, 7.92; N, 15.52.

### Example 20

The title compounds of Examples 1, 16 and 17 were treated by the method of Jakschick et al., Prostaglandins, 16, 733-747 (1978), for their ability to inhibit lepoxygenaise enzyme activity. All compounds were effective at a level of 10 µM.

## Claims (Claims for the following Contracting State(s) : AT, BE, CH, DE, FR, GB, IT, LI, LU, NL, SE)

1. A compound of the formula 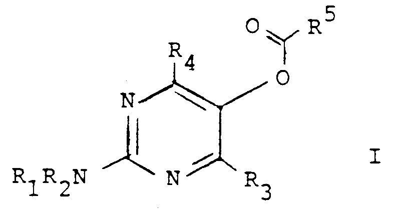 wherein R₁ is hydrogen or (C₁-C₁₅ )alkyl; R₂ is hydrogen, (C₁-C₁₅ )alkyl, cyclopentyl, cyclohexyl, (C₃-Cₗ₅)alkenyl, phenyl, substituted phenyl, (C₇-C₂₀)phenylalkyl, or substituted (C₇-C₂₀)phenylalkyl; or R₁ and R₂ together with the nitrogen atom to which they are attached form a pyrrolidinyl or piperidyl group which may be substituted by one (C₁-C₆)alkyl, phenyl or (C₇-C₂₀)phenylalkyl ; R₃ is (C₁-C₆)alkyl, phenyl, substituted phenyl, (C₇-C₂₀)phenylalkyl, substituted (C₇-C₂₀)phenylalkyl, furyl, thienyl, furyl substituted by one (C₁-C₃)alkyl, or thienyl substituted by one C₁-C₃) alkyl; R₄ is hydrogen, (C₁-C₆)alkyl, phenyl, substituted phenyl, (C₇-C₂₀)phenylalkyl, or substituted (C₇-C₂₀)phenylalkyl; and R₅ is hydrogen, (Ci-Cio) alkyl, (Ci-Cio)alkoxy, phenyl, substituted phenyl, (C₇-C₂₀)phenylalkyl, substituted (C₇-C₂₀)-phenylalkyl, (C₂-C₃)alkylcarboxy, -NR₆R₇, wherein R₆ and R₇ are independently selected from the group consisting of (C₁-C₁₀)alkyl, phenyl, substituted phenyl, (C₇-C₂₀)phenylalkyl and substituted (C₇-C₂ₒ)phenylalkyl; wherein the phenyl moieties on said substituted phenyl and said substituted phenylalkyl may be substituted by one or two moieties selected from the group consisting of fluoro, chloro, (C₁-C₃)alkyl, (C₁-C₃)alkoxy and CF₃; with the proviso that when R₁ and R₂are both hydrogen, R⁵ cannot be methyl and with the further proviso that when R³ and R⁵ are methyl and R⁴ is isopropyl, R¹ and R² are not both methyl and R² is not p-chlorophenylpropyl when R¹ is H; a pharmaceutically acceptable acid addition salt thereof, or, when R₅ is (C₂-C₃)alkylcarboxy, a pharmaceutically acceptable base addition salt thereof.

2. A compound according to claim 1, wherein R₂ is hydrogen, (C₁-C₁₅) alkyl, phenyl, substituted phenyl, (C₇-C₂₀)phenylalkyl, or substituted (C₇-C₂₀)phenylalkyl, wherein the phenyl moieties on said substituted phenyl and said substituted phenylalkyl are substituted by one or two moieties selected from the group consisting of chloro and (C₁-C₃)alkyl; R₃ is (C₁-C₆)alkyl, phenyl, or phenyl substituted by one or two moieties selected from the group consisting of fluoro, chloro, methyl, ethyl, methoxy, ethoxy, and CF₃; and R₄ is hydrogen, (C₁-C₆)alkyl, phenyl or phenyl substituted by one or two moieties selected from the group consisting of methyl and ethyl.

3. A compound according to claim 2, wherein R₁ is hydrogen and R₂ is other than hydrogen.

4. A compound according to claim 1, wherein R¹ is hydrogen; R² is (C₇-C₁₂) phenylalkyl which may be substituted in the phenyl moiety by one or two moieties selected from the group consisting of fluoro, chloro, (C₁-C₃)alkyl, (C₁-C₃)alkoxy, and CF₃; and R₃ and R₄ are each methyl.

5. A compound according to claim 1, wherein R¹ is hydrogen; R₂ is (C₈ -C₉)alkyl, (C₉-C₁₂)phenylalkyl, (C₉-C₁₂) p-chlorophenylalkyl or (C₁₀ -C₁₁ )p-methylphenylalkyl; R₃ and R₄ are each methyl; and R₅ is (C₁-C₆)alkyl or (C₁-C₆)alkoxy.

6. A compound according to claim 1, said compound being selected from the group consisting of 5-acetoxy-4,6-dimethyl-2-phenylhexylaminopyrimidine, 4,6-dimethyl-2-phenylhexylamino-5-(ethyloxyformyl)oxypyrimidine and the acid addition salts of the foregoing compounds.

7. A compound selected from the group consisting of 5-benzoyloxy-4,6-dimethyl-2-phenylhexylaminopyrimidine; 4,6-dimethyl-2-phenylhexylamino-5-o-toluyloxypyrimidine; 5-caproyloxy-4,6-dimethyl-2-phenylhexylaminopyrimidine;5-(5-phenylpentanoyloxy)-4,6-dimethyl-2-phenylhexylaminopyrimidine; 4,6-dimethyl-5-[(p-N,N-diethylaminomethyl)]benzoyloxy-2-phenylhexylamino-pyrimidine; 4,6-dimethyl-2-phenylhexylamino-5-succinoyloxypy rimidine; 4,6-dimethyl-5-[(p-morpholinomethyl)]benzoyloxy-2-phenylhexylaminopyrimidine; 4,6-dimethyl-2-phenylhexylamino-5-propanoyloxypyrimidine; 4,6-dimethyl-2-phenylhexylamino-5-(2-thienoyl)-oxypyrimidine; 4,6-dimethyl-2-phenylhexylamino-5-(phenyloxyformyl)oxypyrimidine; 4,6-dimethyl-2-phenylhexylamino-5-(isobutyloxyformyl)oxypyrimidine; 5-(N,N-dimethylaminoformyl)oxy-4,6-dimethyl-2-phenylhexylaminopyrimidine; 5-(N,N-dimethylaminoformyl)oxy-4-isopropyl-6-methyl-2-phenylhexylaminopyrimidine; 4,6-dimethyl-5-(pivaloyloxy)-2-phenylpentylaminopyrimidine; 4,6-dimethyl-2-phenylpentylamino-5-(isobutyloxyformyl)oxypyrimidine; 5-benzoyloxy-4,6-dimethyl-2-phenylpentylaminopyrimidine; 5-(N,N-dimethylaminoformyl)oxy-4,6-dimethyl-2-phenylpentylaminopyrimidine; and the acid addition salts of the foregoing compounds.

8. A pharmaceutical composition for the treatment of pulmonary, asthmatic, allergic or inflammatory diseases which comprises an amount of a compound according to any one of the preceding claims effective in treating at least one such disease and a pharmaceutically acceptable carrier.

9. A compound according to any one of claims 1 to 7, for use in medicine.

10. Use of a compound according to any one of claims 1 to 7, for making a medicament for treatment of pulmonary, asthmatic, allergic, psoriatic or inflammatory disease.

## Claims (Claims for the following Contracting State(s) : ES, GR)

1. A process for preparing a compound of the formula 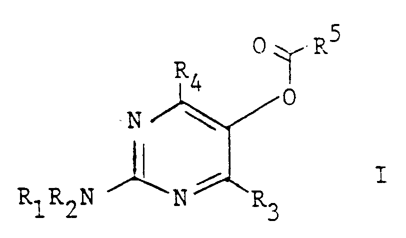 wherein R₁ is hydrogen or (C₁-C₁₅)alkyl; R₂ is hydrogen, (C₁-C₁₅)alkyl, cyclopentyl, cyclohexyl, (C₃-Cₗ₅)alkenyl, phenyl, substituted phenyl, (C₇-C₂₀)phenylalkyl, or substituted (C₇-C₂₀)phenylalkyl; or R₁ and R₂ together with the nitrogen atom to which they are attached form a pyrrolidinyl or piperidyl group which may be substituted by one (C₁-C₆)alkyl, phenyl or (C₇-C₂₀)phenylalkyl ; R₃ is (C₁-C₆)alkyl, phenyl, substituted phenyl, (C₇-C₂₀)phenylalkyl, substituted (C₇-C₂₀)phenylalkyl, furyl, thienyl, furyl substituted by one (C₁-C₃)alkyl, or thienyl substituted by one C₁-C₃) alkyl; R₄ is hydrogen, (C₁-C₆)alkyl, phenyl, substituted phenyl, (C₇-C₂₀)phenylalkyl, or substituted (C₇-C₂₀)phenylalkyl: and R₅ is hydrogen, (Ci-Cio) alkyl, (Ci-Cio)alkoxy, phenyl, substituted phenyl, (C₇-C₂₀)phenylalkyl, substituted (C₇-C₂₀)-phenylalkyl, (C₂-C₃)alkylcarboxy, -NR₆R₇, wherein R₆ and R₇ are independently selected from the group consisting of (C₁-C₁₀)alkyl, phenyl, substituted phenyl, (C₇-C₂₀)phenylalkyl and substituted (C₇-C₂ₒ)phenylalkyl; wherein the phenyl moieties on said substituted phenyl and said substituted phenylalkyl may be substituted by one or two moieties selected from the group consisting of fluoro, chloro, (C₁-C₃)alkyl, (C₁-C₃)alkoxy and CF₃; with the proviso that when R₁ and R₂are both hydrogen, R⁵ cannot be methyl and with the further proviso that when R³ and R⁵ are methyl and R⁴ is isopropyl, R¹ and R² are not both methyl and R² is not p-chlorophenyl when R¹ is H; a pharmaceutically acceptable acid addition salt thereof, or when R₅ is (C₂-C₃)-alkylcarboxy, a pharmaceutically acceptable base addition salt thereof comprising acylating a compound of the formula 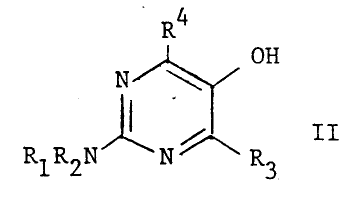 wherein Ri, R₂, R₃ and R₄ are as defined above with an acylating agent, and, if desired, preparing the acid of base addition salt.

2. A process according to claim 1, wherein said acylating agent is an anhydride or an acid chloride.

3. A process according to claim 1, wherein said acylating agent is a compound of the formula 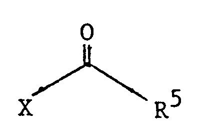 wherein X is chlorine or bromine, and R⁵ is as defined in claim 1.

4. A process according to claim 1, wherein said acylating is a compound of the formula 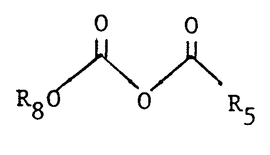 wherein R⁸ is (C₁-C₆' alkyl and R₅ is as defined in claim 1 except that R₅ cannot be alkoxy or aminoalkyl.

5. A process according to any one of claims 1-4, wherein R₂ is hydrogen, (C₁-Cₗ₅)alkyl, phenyl, substituted phenyl, (C₇-C₂₀)phenylalkyl, or substituted (C₇-C₂₀)phenylalkyl, wherein the phenyl moieties on said substituted phenyl and said substituted phenylalkyl are substituted by one or two moieties selected from the group consisting of chloro and (C₁-C₃)alkyl; R₃ is (C₁-C₆)alkyl, phenyl, or phenyl substituted by one or two moieties selected from the group consisting of fluoro, chloro, methyl, ethyl, methoxy, ethoxy, and CF₃; and R₄ is hydrogen, (C₁₋C₆)alkyl, phenyl or phenyl substituted by one or two moieties selected from the group consisting of methyl and ethyl.

6. A process according to claim 5, wherein R₁ is hydrogen and R₂ is other than hydrogen.

7. A process according to any one of claims 1-4, wherein R¹ is hydrogen, R² is (C₇-Cₗ₂)phenylalkyl which may be substituted in the phenyl moiety by one or two moieties selected from the group consisting of fluoro, chloro, (C1-C3)alkyl, (C₁-C₃)alkoxy, and CF₃; and R₃ and R₄ are each methyl.

8. A process according to any one of claims 1-4, wherein R¹ is hydrogen; R₂ is (C₈-C₉)alkyl, (C₉-Cₗ₂)-phenylalkyl, (C₉-C₁₂)p-chlorophenylalkyl or (C₁₀-C₁₃)p-methylphenylalkyl; R₃ and R₄ are each methyl; and R₅ is (C₁-C₆)alkyl or (C₁-C₆ )alkoxy.

9. A process according to claims 1-4, wherein the product is selected from the group consisting of 5-acetoxy-4,6-dimethyl-2-phenylhexylaminopyrimidine, 4,6-dimethyl-2-phenylhexylamino-5-(ethyloxyformyl)oxypyrimidine and the acid addition salts of the foregoing compounds.

10. A process according to any one of claims 1-4, wherein the product is selected from the group consisting of 5-benzoyloxy-4,6-dimethyl-2-phenylhexylaminopyrimidine; 4,6-dimethyl-2-phenylhexylamino-5-o-toluyloxypyrimidine; 5-caproyloxy-4,6-dimethyl-2-phenylhexylaminopyrimidine; 5-(5-phenylpentanoyloxy)-4,6-dimethyl-2-phenylhexylamino-pyrimidine; 4,6-dimethyl-2-phenylhexylamino-5-succinoyloxypyrimidine; 4,6-dimethyl-2-phenylhexyl-amino-5-propanoyloxypyrimidine; 4,6-dimethyl-2-phenylhexylamino-5-(isobutyloxyformyl)-o-xypyrimidine; 5-(N,N-dimethylaminoformyl)oxy-4,6-dimethyl-2-phenylhexylaminopyrimidine; 5-(N,N-dimethylaminoformyl)oxy-4-isopropyl-6-methyl2-phenylhexylaminopyrimidine; 4,6-dimethyl-5-pivaloyloxy-2-phenylpentylaminopyrimidine; 4,6-dimethyl-2-phenylpentylamino-5-(isobutyloxyformyl)oxypyrimidine; 5-benzoyloxy-4,6-dimethyl-2-phenylpentylaminopyrimidine; 5-(N,N-dimethylaminoformyl)oxy-4,6-dimethyl-2-phenylpentylaminopyrimidine; and the acid addition salts of the foregoing compounds.

## Patentansprüche (Patentansprüche für folgende(n) Vertragsstaat(en) : AT, BE, CH, DE, FR, GB, IT, LI, LU, NL, SE)

1. Verbindung der Formel 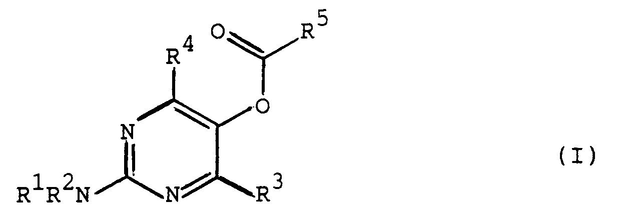 in welcher R¹ Wasserstoff oder C₁₋₅-Alkyl ist; R² Wasserstoff, C₁₋₁₅-Alkyl, Cyclopentyl, Cyclohexyl, C₃-₁₅-Alkenyl, Phenyl, substituiertes Phenyl, C₇-₂₀-Phenylalkyl oder substituiertes C₇-₂₀-Phenylalkyl bedeutet; oder R¹ und R² zusammen mit dem Stickstoffatom, an welches sie gebunden sind, eine Pyrrolidinyl- oder Piperidyl-Gruppe bilden, die durch einen Cᵢ-₆-Alkyl-, Phenyl- oder C₇-₂₀-Phenylalkyl-Rest substituiert sein kann; R³ C₁₋₆-Alkyl, Phenyl, substituiertes Phenyl, C₇₋₂₀-Phenylalkyl, substituiertes C₇-₂₀-Phenylalkyl, Furyl, Thienyl, durch einen C1-3-Alkyl-Rest substituiertes Furyl, oder durch einen C1-3-Alkyl-Rest substituiertes Thienyl ist; R⁴ Wasserstoff, C₁₋₆-Alkyl, Phenyl, substituiertes Phenyl, C₇-₂₀-Phenylalkyl oder substituiertes C₇-₂₀-Phenylalkyl bedeutet; und R⁵ Wasserstoff, C₁₋₁₀-Alkyl, C₁₋₁₀-Alkoxy, Phenyl, substituiertes Phenyl, C₇₋₂₀-Phenylalkyl, substituiertes C₇-₂₀-Phenylalkyl, C₂-₃-Alkylcarboxy, -NR⁶R⁷ ist, worin R⁶ und R⁷, unabhängig voneinander, aus der Gruppe bestehend aus C₁-₁₀-Alkyl, Phenyl, substituiertem Phenyl, C₇₋₂₀-Phenylalkyl und substituiertem C ₇-₂₀-Phenylalkyl ausgewählt sind; worin die Phenyl-Anteile an dem substituierten Phenyl und dem substituierten Phenylalkyl durch einen oder zwei Reste, ausgewählt aus der Gruppe bestehend aus Fluor, Chlor, C₁ -₃-Alkyl, C₁₋₃-Alkoxy und CF₃, substituiert sein können; unter der Bedingung, daß, falls R¹ und R² beide Wasserstoff sind, R⁵ nicht Methyl sein kann und unter der weiteren Bedingung, daß, falls R³ und R⁵ Methyl sind und R⁴ Isopropyl ist, R¹ und R² nicht beide Methyl sind und R² nicht p-Chlorphenylpropyl ist, falls R¹ H ist; ein pharmazeutisch geeignetes Säureadditionssalz davon, oder, falls R⁵ C₂-₃-Alkylcarboxy ist, ein pharmazeutisch geeignetes Basenadditionssalz davon.

2. Verbindung nach Anspruch 1, dadurch gekennzeichnet, daß R² Wasserstoff, Cₗ-₁₅-Alkyl, Phenyl, substituiertes Phenyl, C₇-₂₀-Phenylalkyl, oder substituiertes C₇-₂₀-Phenylalkyl bedeutet, worin die Phenyl-Reste an dem substituierten Phenyl und an dem substituierten Phenylalkyl durch einen oder zwei Reste substituiert sind, ausgewählt aus der Gruppe bestehend aus Chlor und C₁₋₃-Alkyl; R³ C₁₋₆-Alkyl, Phenyl, oder Phenyl, das durch einen oder zwei Reste, ausgewählt aus der Gruppe bestehend aus Fluor, Chlor, Methyl, Ethyl, Methoxy, Ethoxy, und CF₃, substituiert ist, bedeutet; und R⁴ Wasserstoff, C₁-₆-Alkyl, Phenyl oder durch ein oder zwei Reste, ausgewählt aus der Gruppe bestehend aus Methyl und Ethyl, substituiertes Phenyl ist.

3. Verbindung nach Anspruch 2, dadurch gekennzeichnet, daß R¹ Wasserstoff und R² ein anderer Rest als Wasserstoff ist.

4. Verbindung nach Anspruch 1, dadurch gekennzeichnet, daß R¹ Wasserstoff bedeutet; R² C₇-₁₂-Phenylalkyl ist, das in dem Phenyl-Anteil durch einen oder zwei Reste, ausgewählt aus der Gruppe bestehend aus Fluor, Chlor, Cl-3-Alkyl, Cₗ-₃-Alkoxy, und CF₃, substituiert sein kann; und jeder der Reste R³ und R⁴ Methyl ist.

5. Verbindung nach Anspruch 1, dadurch gekennzeichnet, daß R¹ Wasserstoff bedeutet, R² C₈₋₉-Alkyl, C9-12-Phenylalkyl, C₉₋₁₂-p-Chlorphenylalkyl oder C₁₀₋₁₃-p-Methylphenylalkyl ist; jeder der Reste R³ und R⁴ Methyl ist; und R⁵ C₁₋₆-Alkyl oder C₁₋₆-Alkoxy ist.

6. Verbindung nach Anspruch 1, dadurch gekennzeichnet, daß die Verbindung aus der Gruppe bestehend aus 5-Acetoxy-4,6-dimethyl-2-phenylhexylaminopyrimidin, 4,6-Dimethyl-2-phenylhexylamino-5-(ethyloxyformyl)-oxypyrimidin und den Säureadditionssalzen der vorstehenden Verbindungen, ausgewählt ist.

7. Verbindung, dadurch gekennzeichnet, daß sie ausgewählt aus der Gruppe bestehend aus 5-Benzoyloxy-4,6-dimethyl-2-phenylhexylaminopyrimidin; 4,6-Dimethyl-2-phenylhexylamino-5-o-toluylox- ypyrimidin; 5-Caproyloxy-4,6-dimethyl-2-phenylhexylaminopyrimidin; 5-(5-Phenylpentanoyloxy)-4,6-dimethyl-2-phenylhexylaminopyrimidin; 4,6-Dimethyl-5-[(p-N,N-Diethylaminomethyl)]-benzoyloxy-2-phe- nylhexylaminopyrimidin; 4,6-Dimethyl-2-phenylhexylamino-5-succinoyloxypyrimidin; 4,6-Dimethyl-5-[(p-Morpholinomethyl)]-benzoyloxy-2-phenylhexylaminopyrimidin; 4,6-Dimethyl-2-phenylhexylamino-5-pro- panoyloxypyrimidin; 4,6-Dimethyl-2-phenylhexylamino-5-(2-thienoyl)-oxypyrimidin; 4,6-Dimethyl-2-phenylhexylamino-5-(phenyloxyformyl)oxypyrimidin; 4,6-Dimethyl-2-phenylhexylamino-5-(isobutyloxyformyl)-oxypyrimidin; 5-(N,N-Dimethylaminoformyl)-oxy-4,6-dimethyl-2-phenylhexylamino- pyrimidin; 5-(N,N-Dimethylaminoformyl)-oxy-4-isopropyl-6-methyl-2-phenylhexylaminopyrimidin; 4,6-Dimethyl-5-(pivaloyloxy)-2-phenylpentylaminopyrimidin; 4,6-Dimethyl-2-phenylpentylamino-5-(isobutyloxyformyl)-oxypyrimidin; 5-Benzoyloxy-4,6-dimethyl-2-phenylpentylaminopyrimidin; 5-(N,N-Di- methylaminoformyl)-oxy-4,6-dimethyl-2-phenylpentylaminopyrimidin; und die Säureadditionssalze der vorstehenden Verbindungen.

8. Pharmazeutische Zusammensetzung zur Behandlung von pulmonalen, asthmatischen, allergischen oder entzündlichen Krankheiten, dadurch gekennzeichnet, daß sie eine Menge einer Verbindung gemäß irgendeinem der vorstehenden Ansprüche, wirksam zur Behandlung von zumindest einer derartigen Krankheit, und einen pharmazeutisch verträglichen Träger, enthält.

9. Verbindung nach irgendeinem der Ansprüche 1 bis 7 für die Verwendung in der Medizin.

10. Die Verwendung einer Verbindung nach irgendeinem der Ansprüche 1 bis 7 zur Herstellung eines Medikaments zur Behandlung einer pulmonalen, asthmatischen, allergischen, psoriatischen oder entzündlichen Krankheit.

## Patentansprüche (Patentansprüche für folgende(n) Vertragsstaat(en) : ES, GR)

1. Verfahren zur Herstellung einer Verbindung der Formel 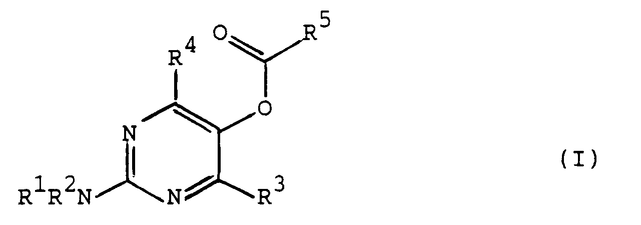 in welcher R¹ Wasserstoff oder Cₗ-₁₅-Alkyl ist; R² Wasserstoff, Cₗ-₁₅-Alkyl, Cyclopentyl, Cyclohexyl, C₃-₁₅-Alkenyl, Phenyl, substituiertes Phenyl, C₇-₂₀-Phenylalkyl oder substituiertes C₇-₂₀-Phenylalkyl bedeutet; oder R¹ und R² zusammen mit dem Stickstoffatom, an welches sie gebunden sind, eine Pyrrolidinyl- oder Piperidyl-Gruppe bilden, die durch einen Cᵢ-₆-Alkyl-, Phenyl- oder C₇-₂₀-Phenylalkyl-Rest substituiert sein kann; R³ C₁₋₆-Alkyl, Phenyl, substituiertes Phenyl, C₇₋₂₀-Phenylalkyl, substituiertes C₇-₂₀-Phenylalkyl, Furyl, Thienyl, durch einen C1-3-Alkyl-Rest substituiertes Furyl, oder durch einen C1-3-Alkyl-Rest substituiertes Thienyl ist; R⁴ Wasserstoff, C₁₋₆-Alkyl, Phenyl, substituiertes Phenyl, C₇-₂₀-Phenylalkyl oder substituiertes C₇-₂₀-Phenylalkyl bedeutet; und R⁵ Wasserstoff, C₁₋₁₀-Alkyl, C₁₋₁₀-Alkoxy, Phenyl, substituiertes Phenyl, C₇₋₂₀-Phenylalkyl, substituiertes C₇-₂₀-Phenylalkyl, C₂-₃-Alkylcarboxy, -NR⁶R⁷ ist, worin R⁶ und R⁷, unabhängig voneinander, aus der Gruppe bestehend aus C₁₋₁₀-Alkyl, Phenyl, substituiertem Phenyl, C₇₋₂₀-Phenylalkyl und substituiertem C ₇-₂₀-Phenylalkyl ausgewählt sind; worin die Phenyl-Anteile an dem substituierten Phenyl und dem substituierten Phenylalkyl durch einen oder zwei Reste, ausgewählt aus der Gruppe bestehend aus Fluor, Chlor, C₁₋₃-Alkyl, C₁-C₃-Alkoxy und CF₃, substituiert sein können; unter der Bedingung, daß, falls R¹ und R² beide Wasserstoff sind, R⁵ nicht Methyl sein kann und unter der weiteren Bedingung, daß, falls R³ und R⁵ Methyl sind und R⁴ Isopropyl ist, R¹ und R² nicht beide Methyl sind und R² nicht p-Chlorphenylpropyl ist, falls R¹ H ist; ein pharmazeutisch geeignetes Säureadditionssalz davon, oder, falls R⁵ C₂-₃-Alkylcarboxy ist, ein pharmazeutisch geeignetes Basenadditionssalz davon, dadurch gekennzeichnet, daß es das Acylieren einer Verbindung der Formel 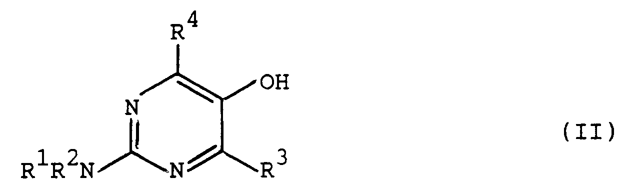 in welcher R¹, R², R³ und R⁴ die gleiche Bedeutung wie oben besitzen, mit einem Acylierungsmittel, und, falls gewünscht, das Herstellen der Säure des Basenadditionssalzes, umfaßt.

2. Verfahren nach Anspruch 1, dadurch gekennzeichnet, daß das Acylierungsmittel ein Anhydrid oder ein Säurechlorid ist.

3. Verfahren nach Anspruch 1, dadurch gekennzeichnet, daß das Acylierungsmittel eine Verbindung der Formel 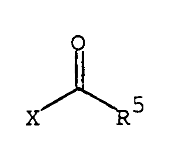 ist, in welcher X Chlor oder Brom bedeutet und R⁵ die gleiche Bedeutung wie in Anspruch 1 besitzt.

4. Verfahren nach Anspruch 1, dadurch gekennzeichnet, daß das Acylierungsmittel eine Verbindung der Formel 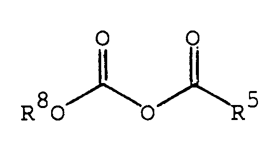 ist, in welcher R⁸ Ci-6-Alkyl ist und R⁵ die gleiche Bedeutung wie in Anspruch 1 besitzt, mit der Ausnahme, daß R⁵ nicht Alkoxy oder Aminoalkyl sein kann.

5. Verfahren nach einem der Ansprüche 1 bis 4, dadurch gekennzeichnet, daß R² Wasserstoff, C₁₋₁₅-Alkyl, Phenyl, substituiertes Phenyl, C₇-₂₀-Phenylalkyl, oder substituiertes C₇-₂₀-Phenylalkyl bedeutet, worin die Phenyl-Reste an dem substituierten Phenyl und an dem substituierten Phenylalkyl durch einen oder zwei Reste substituiert sind, ausgewählt aus der Gruppe bestehend aus Chlor und C₁₋₃-Alkyl; R³ C₁₋₆-Alkyl, Phenyl, oder Phenyl, das durch einen oder zwei Reste, ausgewählt aus der Gruppe bestehend aus Fluor, Chlor, Methyl, Ethyl, Methoxy, Ethoxy, und CF₃, substituiert ist, bedeutet; und R⁴ Wasserstoff, C₁₋₆-Alkyl, Phenyl oder durch ein oder zwei Reste, ausgewählt aus der Gruppe bestehend aus Methyl und Ethyl, substituiertes Phenyl ist.

6. Verfahren nach Anspruch 5, dadurch gekennzeichnet, daß R¹ Wasserstoff und R² ein anderer Rest als Wasserstoff ist.

7. Verfahren nach einem der Ansprüche 1 bis 4, dadurch gekennzeichnet, daß R¹ Wasserstoff bedeutet; R² C₇-₁₂-Phenylalkyl ist, das in dem Phenyl-Anteil durch einen oder zwei Reste, ausgewählt aus der Gruppe bestehend aus Fluor, Chlor, C₁₋₃-Alkyl, C₁₋₃-Alkoxy, und CF₃, substituiert sein kann; und jeder der Reste R³ und R⁴ Methyl ist.

8. Verfahren nach einem der Ansprüche 1 bis 4, dadurch gekennzeichnet, daß R¹ Wasserstoff bedeutet; R² C₈₋₉-Alkyl, C9-12-Phenylalkyl, C₉₋₁₂-p-Chlorphenylalkyl oder C₁₀₋₁₃-p-Methylphenylalkyl ist; jeder der Reste R³ und R⁴ Methyl bedeutet; und R⁵ C₁₋₆-Alkyl oder C₁₋₆-Alkoxy ist.

9. Verfahren nach einem der Ansprüche 1 bis 4, dadurch gekennzeichnet, daß das Produkt aus der Gruppe bestehend aus 5-Acetoxy-4,6-dimethyl-2-phenylhexylaminopyrimidin, 4,6-Dimethyl-2-phenylhexylamino-5-(ethyloxyformyl)-oxypyrimidin und den Säureadditionssalzen der vorstehenden Verbindungen, ausgewählt ist.

10. Verfahren nach einem der Ansprüche 1 bis 4, dadurch gekennzeichnet, daß das Produkt ausgewählt ist aus der Gruppe bestehend aus 5-Benzoyloxy-4,6-dimethyl-2-phenylhexylaminopyrimidin; 4,6-Dimethyl-2-phenylhexylamino-5-o-toluyloxypyrimidin; 5-Caproyloxy-4,6-dimethyl-2-phenylhexylaminopy- rimidin; 5-(5-Phenylpentanoyloxy)-4,6-dimethyl-2-phenylhexylaminopyrimidin; 4,6-Dimethyl-2-phenylhexylamino-5-succinoyloxypyrimidin; 4,6-Dimethyl-2-phenylhexylamino-5-propanoyloxypyrimidin; 4,6-Dimethyl-2-phenylhexylamino-5-(isobutyloxyformyl)-oxypyrimidin; 5-(N,N-Dimethylaminoformyl)-oxy4,6-dimethyl-2-phenylhexylaminopyrimidin; 5-(N,N-Dimethylaminoformyl)-oxy-4-isopropyl-6-methyl-2-phenylhexylaminopyrimidin; 4,6-Dimethyl-5-pivaloyloxy-2-phenylpentylaminopyrimidin; 4,6-Dimethyl-2-phenylpentylamino-5-(isobutyloxyformyl)-oxypyrimidin; 5-Benzoyloxy-4,6-dimethyl-2-phenylpentylami- nopyrimidin; 5-(N,N-Dimethylaminoformyl)-oxy-4,6-dimethyl-2-phenylpentylaminopyrimidin; und die Säureadditionssalze der vorstehenden Verbindungen.

## Revendications (Revendications pour l'(les) Etat(s) contractant(s) suivant(s) : AT, BE, CH, DE, FR, GB, IT, LI, LU, NL, SE)

1. Composé de formule 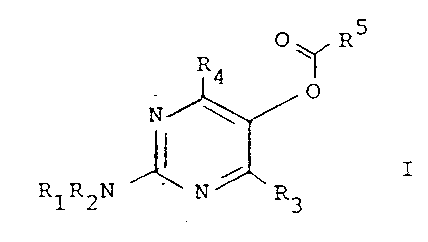 dans laquelle R₁ est l'hydrogène ou un groupe alkyle en Ci à C₁₅ ; R₂ est l'hydrogène, un groupe alkyle en Ci à C₁₅, cyclopentyle, cyclohexyle, alcényle en C₃ à C₁₅, phényle, phényle substitué, phénylalkyle en C₇ à C₂₀ ou phénylalkyle en C₇ à C₂₀ substitué ; ou bien R₁ et R₂ forment, conjointement avec l'atome d'azote auquel ils sont liés, un groupe pyrrolidinyle ou pipéridyle qui peut être substitué par un radical alkyle en Ci à C₆, phényle ou phénylalkyle en C₇ à C₂₀ ; R₃ est un groupe alkyle en Ci à C₆, phényle, phényle substitué, phénylalkyle en C₇ à C₂₀, phénylalkyle en C₇ à C₂₀ substitué, furyle, thiényle, furyle substitué par un radical alkyle en Ci à C₃ ou thiényle substitué par un radical alkyle en Ci à C₃ ; R₄ est l'hydrogène, un groupe alkyle en Ci à C₆, phényle, phényle substitué, phénylalkyle en C₇ à C₂₀ ou phénylalkyle en C₇ à C₂₀ substitué : et R₅ est l'hydrogène, un groupe alkyle en Ci à C₁₀, alkoxy en Ci à C₁₀, phényle, phényle substitué, phénylalkyle en C₇ à C₂₀, phénylalkyle en C₇ à C₂₀ substitué, alkylcarboxy en C₂ ou C₃, -NR₆R₇, où R₆ et R₇ sont choisis indépendamment dans le groupe comprenant un radical alkyle en Ci à Cio, phényle, phényle substitué, phénylalkyle en C₇ à C₂₀ et phénylalkyle en C₇ à C₂₀ substitué ; les groupements phényle dudit phényle substitué et dudit phénylalkyle substitué peuvent être substitués par un ou deux radicaux choisis dans le groupe des radicaux fluoro, chloro, alkyle en Ci à C₃, alkoxy en Ci à C₃ et CF₃ ; sous réserve que lorsque R₁ et R₂ sont tous deux de l'hydrogène, R⁵ ne puisse pas être un groupe méthyle et sous réserve en outre que lorsque R³ et R⁵ sont des groupes méthyle et que R⁴ est un groupe isopropyle, R¹ et R² ne soient pas tous deux des groupes méthyle et que R² ne soit pas un groupe p-chlorophénylpropyle lorsque R¹ est l'hydrogène ; un sel d'addition d'acide pharmaceutiquement acceptable de ce composé ou bien, lorsque R₅ est un groupe alkylcarboxy en C₂ ou C₃, un sel d'addition de base pharmaceutiquement acceptable de ce composé.

2. Composé suivant la revendication 1, dans lequel R₂ est l'hydrogène, un groupe alkyle en Ci à C₁₅, phényle, phényle substitué, phénylalkyle en C₇ à C₂₀ ou phénylalkyle en C₇ à C₂₀ substitué, les groupements phényle dudit groupe phényle substitué et dudit groupe phénylalkyle substitué étant substitués par un ou deux radicaux choisis dans le groupe des radicaux chloro et alkyle en Ci à C₃ ; R₃ est un groupe alkyle en Ci à C₆, phényle ou phényle substitué par un ou deux radicaux choisis dans le groupe des radicaux fluoro, chloro, méthyle, éthyle, méthoxy, éthoxy et CF₃ : et R₄ est l'hydrogène, un groupe alkyle en Ci à C₆, phényle ou phényle substitué par un ou deux radicaux choisis entre les radicaux méthyle et éthyle.

3. Composé suivant la revendication 2, dans lequel R₁ est l'hydrogène et R₂ est autre chose que l'hydrogène.

4. Composé suivant la revendication 1, dans lequel R¹ est l'hydrogène ; R² est un groupe phénylalkyle en C₇ à C₁₂ qui peut être substitué dans le groupement phényle par un ou deux radicaux choisis dans le groupe des radicaux fluoro, chloro, alkyle en Ci à C₃, alkoxy en Ci à C₃ et CF₃ ; et R₃ et R₄ représentent chacun un groupe méthyle.

5. Composé suivant la revendication 1, dans lequel R¹ est l'hydrogène ; R₂ est un groupe alkyle en C₈ ou C₉, phénylalkyle en C₉ à C₁₂ et p-chlorophénylalkyle en C₃ à C₁₂ ou p-méthylphénylalkyle en C₁₀ à à C₁₃ ; R₃ et R₄ représentent chacun un groupe méthyle ; et R₅ est un groupe alkyle en Ci à C₆ ou alkoxy en Ci à C₆.

6. Composé suivant la revendication 1, choisi dans le groupe comprenant la 5-acétoxy-4,6-diméthyl-2- phénylhexylaminopyrimidine, la 4,6-diméthyl-2-phénylhexylamino-5-(éthyloxyformyl)oxypyrimidine et les sels d'addition d'acides des composés ci-dessus.

7. Composé choisi dans le groupe comprenant la 5-benzoyloxy-4,6-diméthyl-2-phénylhexylaminopyrimidi- ne ; la 4,6-diméthyl-2-phénylhexylamino-5-o-toluyloxypyrimidine ; la 5-caproyloxy-4,6-diméthyl-2-phé- nylhexylaminopyrimidine ; la 5-(5-phénylpentanoyloxy)-4,6-diméthyl-2-phénylhexylaminopyrimidine ; la 4,6-diméthyl-5-[(p-N,N-diéthylaminométhyl)]benzoyloxy-2-phénylhexylaminopyrimidine ; la 4,6-diméthyl-2-phénylhexylamino-5-succinoyloxypyrimidine ; la 4,6-diméthyl-5-[(p-morpholinométhyl)]benzoyloxy-2- phénylhexylaminopyrimidine ; la 4,6-diméthyl-2-phénylhexylamino-5-propanoyloxypyrimidine ; la 4,6-diméthyl-2-phénylhexylamino-5-(2-thiénoyl)oxypyrimidine _{;} la 4,6-diméthyl-2-phénylhexylamino-5-(phényloxyformyl)oxypyrimidine _{;} la 4,6-diméthyl-2-phénylhexylamino-5-(isobutyloxyformyl)-oxypyrimidine; la 5-(N,N-diméthylaminoformyl)oxy-4,6-diméthyl-2-phénylhexylaminopyrimidine ; la 5-(N,N-diméthylaminoformyl)oxy-4-isopropyl-6-méthyl-2-phénylhexylaminopyrimidine ; la 4,6-diméthyl-5-(pivaloyloxy)-2-phénylpentylaminopyrimidine _{;} la 4,6-diméthyl-2-phénylpentylamino-5-(isobutyloxyformyl)-oxypyrimidine ; la 5-benzoyloxy-4,6-diméthyl-2-phénylpentylaminopyrimidine ; la 5-(N,N-diméthylaminoformyl)oxy-4,6-diméthyl-2-phénylpentylaminopyrimidine et les sels d'addition d'acides des composés ci-dessus.

8. Composition pharmaceutique destinée au traitement de troubles pulmonaires, asthmatiques, allergiques ou inflammatoires, qui comprend une quantité d'un composé suivant l'une quelconque des revendications précédentes, efficace dans le traitement d'au moins l'une de ces maladies, et un support acceptable du point de vue pharmaceutique.

9. Composé suivant l'une quelconque des revendications 1 à 7, destiné à être utilisé en médecine.

10. Utilisation d'un composé suivant l'une quelconque des revendications 1 à 7, pour la préparation d'un médicament destiné au traitement d'un trouble pulmonaire, asthmatique, allergique, psoriasique ou inflammatoire.

## Revendications (Revendications pour l'(les) Etat(s) contractant(s) suivant(s) : ES, GR)

1. Procédé de production d'un composé de formule 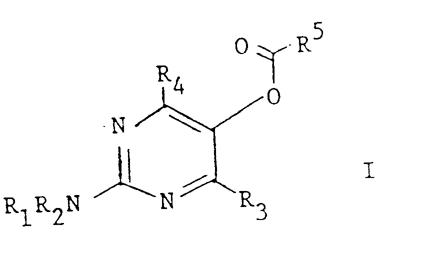 dans laquelle R₁ est l'hydrogène ou un groupe alkyle en Ci à C₁₅ ; R₂ est l'hydrogène, un groupe alkyle en Ci à C₁₅, cyclopentyle, cyclohexyle, alcényle en C₃ à C₁₅, phényle, phényle substitué, phénylalkyle en C₇ à C₂₀ ou phénylalkyle en C₇ à C₂₀ subsituté ; ou bien R₁ et R₂ forment, conjointement avec l'atome d'azote auquel ils sont liés, un groupe pyrrolidinyle ou pipéridyle qui peut être substitué par un radical alkyle en Ci à C₆, phényle ou phénylalkyle en C₇ à C₂₀ ; R₃ est un groupe alkyle en Ci à C₆, phényle, phényle substitué, phénylalkyle en C₇ à C₂₀, phénylalkyle en C₇ à C₂₀ substitué, furyle, thiényle, furyle substitué par un radical alkyle en Ci à C₃ ou thiényle substitué par un radical alkyle en C₁ à C₃ ; R₄ est l'hydrogène, un groupe alkyle en Ci à C₆, phényle, phényle substitué, phénylalkyle en C₇ à C₂₀ ou phénylalkyle en C₇ à C₂₀ substitué ; et R₅ est l'hydrogène, un groupe alkyle en Ci à C₁₀, alkoxy en Ci à C₁₀, phényle, phényle substitué, phénylalkyle en C₇ à C₂₀, phénylalkyle en C₇ à C₂₀ substitué, alkylcarboxy en C₂ ou C₃, -NR₆R₇, où R₆ et R₇ sont choisis indépendamment dans le groupe comprenant un radical alkyle en Ci à Cio, phényle, phényle substitué, phénylalkyle en C₇ à C₂₀ et phénylalkyle en C₇ à C₂₀ substitué ; les groupements phényle dudit phényle substitué et dudit phénylalkyle substitué peuvent être substitués par un ou deux radicaux choisis dans le groupe des radicaux fluoro, chloro, alkyle en Ci à C₃, alkoxy en Ci à C₃ et CF₃ ; sous réserve que lorsque R₁ et R₂ sont tous deux de l'hydrogène, R⁵ ne puisse pas être un groupe méthyle et sous réserve en outre que lorsque R³ et R⁵ sont des groupes méthyle et que R⁴ est un groupe isopropyle, R¹ et R² ne soient pas tous deux des groupes méthyle et que R² ne soit pas un groupe p-chlorophényle lorsque R¹ est l'hydrogène ; d'un sel d'addition d'acide pharmaceutiquement acceptable de ce composé ou, lorsque R₅ est un groupe alkylcarboxy en C₂ ou C₃, d'un sel d'addition de base acceptable du point de vue pharmaceutique de ce composé, qui consiste à acyler un composé de formule : 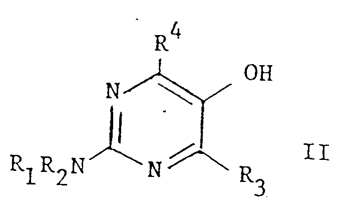 dans laquelle Ri, R₂, R₃ et R₄ sont tels que définis ci-dessus, avec un agent acylant et, le cas échéant, à préparer le sel d'addition d'acide ou de base.

2. Procédé suivant la revendication 1, dans lequel l'agent acylant est un anhydride ou un chlorure d'acide.

3. Procédé suivant la revendication 1, dans lequel l'agent acylant est un composé de formule 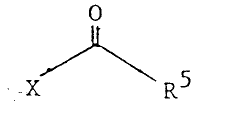 dans laquelle X est le chlore ou le brome et R⁵ est tel que défini dans la revendication 1.

4. Procédé suivant la revendication 1, dans lequel le composé acylant est un composé de formule 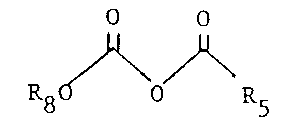 dans laquelle R⁸ est un groupe alkyle en Ci à C₆ et R₅ est tel que défini dans la revendication 1, excepté que R₅ ne peut pas être un radical alkoxy ou aminoalkyle.

5. Procédé suivant l'une quelconque des revendications 1 à 4, dans lequel R₂ est l'hydrogène, un groupe alkyle en Ci à C₁₅, phényle, phényle substitué, phénylalkyle en C₇ à C₂₀ ou phénylalkyle en C₇ à C₂₀ substitué, les groupements phényle dudit groupe phényle substitué et dudit groupe phénylalkyle substitué étant substitués par un ou deux radicaux choisis dans le groupe des radicaux chloro et alkyle en Ci à C₃ ; R₃ est un groupe alkyle en Ci à C₆, phényle ou phényle substitué par un ou deux radicaux choisis dans le groupe des radicaux fluoro, chloro, méthyle, éthyle, méthoxy, éthoxy et CF₃ ; et R₄ est l'hydrogène, un groupe alkyle en Ci à C₆, phényle ou phényle substitué par un ou deux radicaux choisis entre les radicaux méthyle et éthyle.

6. Procédé suivant la revendication 5, dans lequel R₁ est l'hydrogène et R₂ représente autre chose que l'hydrogène.

7. Procédé suivant l'une quelconque des revendications 1 à 4, dans lequel R¹ est l'hydrogène, R² est un groupe phénylalkyle en C₇ à C₁₂ qui peut être substitué dans le groupement phényle par un ou deux radicaux choisis dans le groupe des radicaux fluoro, chloro, alkyle en Ci à C₃, alkoxy en Ci à C₃ et CF₃ ; et R³ et R⁴ représentent chacun un groupe méthyle.

8. Procédé suivant l'une quelconque des revendications 1 à 4, dans lequel R¹ est l'hydrogène ; R₂ est un groupe alkyle en C₈ ou C₉, un groupe phénylalkyle en C₉ à C₁₂, un groupe p-chlorophénylalkyle en C₉ à C₁₂ ou un groupe p-méthylphénylalkyle en C₁₀ à C₁₃ ; R₃ et R₄ sont chacun un groupe méthyle ; et R₅ est un groupe alkyle en Ci à C₆ ou alkoxy en Ci à C₆.

9. Procédé suivant les revendications 1 à 4, dans lequel le produit est choisi dans le groupe comprenant la 5-acétoxy-4,6-diméthyl-2-phénylhexylaminopyrimidine, la 4,6-diméthyl-2-phénylhexylamino-5-(éthyloxyformyl)oxypyrimidine et les sels d'addition d'acides des composés ci-dessus.

10. Procédé suivant l'une quelconque des revendications 1 à 4, dans lequel le produit est choisi dans le groupe comprenant la 5-benzoyloxy-4,6-diméthyl-2-phénylhexylaminopyrimidine ; la 4,6-diméthyl-2- phénylhexylamino-5-o-toluyloxypyrimidine ; la 5-caproyloxy-4,6-diméthyl-2-phénylhexylaminopyrimidine _{;} la 5-(5-phénylpentanoyloxy)-4,6-diméthyl-2-phénylhexylaminopyrimidine _{;} la 4,6-diméthyl-2- phénylhexylamino-5-succinoyloxypyrimidine ; la 4,6-diméthyl-2-phénylhexylamino-5-propanoyloxy-pyrimidine ; la 4,6-diméthyl-2-phénylhexylamino-5-(isobutyloxyformyl)oxypyrimidine; la 5-(N,N-diméthylami- noformyl)oxy-4,6-diméthyl-2-phénylhexylaminopyrimidine _{;} la 5-(N,N-diméthylaminoformyl)oxy-4-isopropyl-6-méthyl-2-phénylhexylaminopyrimidine ; la 4,6-diméthyl-5-pivaloyloxy-2-phénylpentylamino- pyrimidine ; la 4,6-diméthyl-2-phénylpentylamino-5-(isobutyloxyformyl)oxypyrimidine ; la-5-benzoyloxy-4,6-diméthyl-2-phénylpentylaminopyrimidine ; la 5-(N,N-diméthylaminoformyl)oxy-4,6-diméthyl-2-phé- nylpentylaminopyrimidine ; et les sels d'addition d'acides des composés ci-dessus.
